# EUROPEAN PATENT SPECIFICATION

(11) **EP 2 316 514 B1**
(45) Date of publication and mention of the grant of the patent: **16.10.2013**
(21) Application number: 10189308.9
(22) Date of filing: 28.10.2010
(51) Int. Cl.: A61H 9/00, A61M 11/02, A61M 16/16, A61M 16/00, A61M 16/08, A61M 11/06, A61M 16/12

(54) **Composite lung therapy device**
Lungentherapievorrichtung aus Verbundstoff
Dispositif composite de thérapie des poumons

(30) Priority: 02.11.2009 US 257350 P
(43) Date of publication of application: 04.05.2011
(73) Proprietor: Comedica Incorporated, Dallas, Texas 75214 (US)
(72) Inventor: Faram, Joseph Dee, Dallas, TX 75214 (US)
(74) Representative: Findlay, Alice Rosemary

(56) References cited:
- WO-A1-94/20060
- WO-A1-98/17224
- US-A- 4 977 889
- US-A1- 2008 066 754

## Description

The present disclosure relates, in general, to a respiratory therapy device. More specifically, the present disclosure relates to a device for providing a composite breathing treatment for the purposes of clearing secretions from the lungs, expanding the airways, and delivering medicated aerosol.

Atelectasis is the partial or total collapse of the lung. Although this condition may occur as a result of pressure being exerted from outside the lungs by such maladies as a tumor or fluid buildup in the pleural space, it is most often caused by obstruction within the airways. When blockage develops, the air in the small air sacs, or alveoli, on the distal side of the obstruction is absorbed into the bloodstream and the air sacs become diminished in size or collapse. These alveoli then often fill with blood cells, mucus, or serum, making them highly susceptible to infection. Atelectasis may happen suddenly or may gradually manifest over a long period of time. In either case, the disorder may lead to shortness of breath, decreased oxygen levels, increased heart rate, and infection, which in turn, can result in outcomes ranging from simple discomfort to death.

Traditionally, prevention and treatment of atelectasis has included a wide variety of devices that facilitate treatment in three main areas: 1) medicated aerosol delivery, 2) lung expansion therapy, and 3) secretion clearance therapy. The variety of devices used in these therapies present a number of problems. First, for any given patient it is difficult to know in advance which therapy or combination of therapies is most appropriate. After assessing the patient at bedside, the clinician may decide that the patient requires a different therapy than planned, at which point he or she may need to return to a supply room to secure the proper therapy device or devices. This can be time consuming and can delay treatment at a time when prompt application of treatment is desirable. Second, in order to be prepared to deliver appropriate therapy, the healthcare provider may need to stock a number of devices, which presents the provider with the requirements of storage space and the need to deal with a number of suppliers. Furthermore, maintaining a number of different devices, and their attendant disposable accessories, to provide multiple therapy options increases costs to the healthcare provider and ultimately the patient. Third, in order to adequately operate various devices, a clinician may need to attend multiple training sessions, further increasing costs to the healthcare provider and patient.

An additional drawback found with known existing devices is that no single device is believed to be capable of delivering aerosol, lung expansion, and secretion clearance therapies concurrently or contiguously, even though such uses may enhance respiratory therapy. For example, if airway oscillation is administered externally with a circumferential chest band that supplies a compressive oscillating pulse to the chest wall, concurrent positive pressure lung expansion therapy delivered internally to the airways may enhance the secretion clearance effects of the external oscillation. Additionally, simultaneous medicated aerosol Included in the therapy mix could enhance the efficacy of both airway oscillation and airway expansion. If airway oscillation is administered internally by means of pulsatile positive pressure, it would be beneficial to seamlessly change the therapy mode to airway expansion without interruption in order to prevent possible collapse of airways between therapy modes. As in the previous example, medicated aerosol delivered during both the internal airway oscillation therapy and the airway expansion therapy could augment both therapies.
Specific examples of existing devices can be found in US 2008/0066754, US 4,977,889, WO 94/20060 and WO 98/17224.
US 2008/0066754 discloses a continuous high-frequency oscillation breathing device that delivers therapy during both inhalation and exhalation in order to assist in clearing secretions from the lungs. The device comprises a venturi patient interface circuit In combination with a medicated aerosol to deliver the high-frequency oscillation therapy.
US 4,977,889 describes an apparatus for pummeling of the thoracic cavity for the purpose of aiding respiration, The apparatus comprises a vest to be worn by the patient. The vest is connected to an alternating compression machine for providing alternating pressure to the vest in order to provide a mechanical thumping to the patient's thoracic cavity.
WO 94/20060 describes an active compression/decompression CPR device capable of providing both complete cardiopulmonary support and cardiopulmonary assistance. The device includes two or more thoracic compressors, such as inflatable bladders.
WO 98/17224 describes an apparatus for resuscitating the cardiac/pulmonary activity of a patient that includes the use of pneumatically controlled inflatable/deflatable cuffs secured over the patient's legs and abdomen, and optionally the chest. The cuffs may be inflated and deflated at a cyclical rate of 10-40 cycles/min. In addition, a separate ventilator and face mask may be provided to supply breathable air to the patient.

The present invention provides an apparatus, that has any one or more of the following features alone or in any combination.

The apparatus comprises a gas control box having a source of gas operable to provide a substantially continuous positive gas flow from the gas control box (100) through a first port (128, 130) of the gas control box, a regulator to regulate the flow of said substantially continuous positive gas flow between said source of gas and a patient, and a selector to select between modes of operation, wherein said modes of operation comprise at least a first mode of operation and a second mode of operation by which different respiratory therapies are provided to the patient's lungs through the patient's mouth; a patient Interface circuit coupled to the source of gas of the gas control box via the first output, the patient interface circuit having a breathing head assembly through which the patient exhales and Inhales, the breathing head assembly having at least one aperture open to the ambient to allow ingress and egress of flow, wherein said at least one aperture is calibrated to allow patient exhalation and prevent stacking of successive volumes of gas in the airway of the patient, the breathing head assembly having a venturi tube fluidly connected to said at least one aperture, and an adjuster to partially occluding said at least one aperture; and at least one Inflatable bladder coupled to the gas control box through a second port of the gas control box and placed adjacent a patient's chest, the at least on inflatable bladder being repetitively inflated and deflated by the gas control box.

An apparatus according to the present disclosure may operate to deliver high frequency airway oscillation, continuous positive airway pressure (CPAP), and aerosol therapy either concurrently or contiguously. Such an apparatus, therefore, combines aerosol delivery, lung expansion therapy, and secretion clearance therapy into a single device and allows a method of treatment which combines multiple treatments in either a concurrent fashion or immediately proximal to one another.

In one mode, aerosol delivery can be combined with either pulsatile positive gas flow internal to the patient's airways to provide a secretion clearance mode, or with a continuous positive gas flow to provide a lung expansion mode. A third mode of operation is also disclosed in which linear gas flow without aerosol delivery may be provided to the patient. Further, the pulsatile gas flow can be connected to the bladder(s) of a chest band that fits circumferentially around the patient's chest in order to supply a compressive oscillating pulse to the chest wall. The oscillating pulse to the chest wall may provide external airway oscillation.

Some embodiments may comprise a gas control box, a patient interface circuit, and a chest wall band. The gas control box may control the flow of gas from the gas source to the patient interface circuit and/or the chest wall band. The patient interface circuit may be of single patient use, may use a fixed venture, and may have at least one orifice open to the atmosphere for entrainment. The patient interface circuit may also Include at least one exhalation opening which can be adjusted to maintain a positive pressure in the lungs at the end of exhalation (Positive End Expiratory Pressure or PEEP) without the stacking of successive volumes of gas in the airways (breath stacking). PEEP helps to open the airways and keep them open during the therapy.

The chest wall band may contain a bladder or multiple bladders and may wrap circumferentially around the patient's chest. The chest wall band may be single patient use. The apparatus may be designed to minimize both clinical training requirements and operator errors during treatment. Additionally, with several therapies combined into one machine, the clinician can change therapies near instantly as needed without having to return to an equipment storage area to retrieve other devices. Storage requirements are reduced and the healthcare provider can deal with a single source vender for supplies, training, and repairs. It is expected that the healthcare provider would also achieve significant cost savings through the reduction in the number of different types of equipment required to perform the various treatments performed by the current apparatus.

Among the therapies or respiratory treatments that may be possible by using the apparatuses of the present disclosure include provision of aerosolized medicament, supply of a continuous positive gas flow, and supply of a pulsating continuous gas flow. In some contemplated embodiments, respiratory treatment if provided by an apparatus incorporating a patient interface having a venturi tube in connection with at least one aperture open to the ambient that allow ingress and egress of flow.

Also according to this disclosure, respiratory treatment is provided by an apparatus incorporating a chest wall band for administering compressive oscillating pulses to the chest wall. The chest wall band may administer compressive oscillating pulses to the chest wall and a patient interface may be operable to provide aerosolized medicament, such as via a nebulizer. Alternatively or additionally, the chest wall band may administer compressive oscillating pulses to the chest wall and the patient interface may be operable to provide linear gas flow. Further alternatively or additionally, the chest wall band may administer compressive oscillating pulses to the chest wall and the patient interface may be operable to provide either a linear gas flow or a pulsating gas flow. Thus, according to the present disclosure, respiratory treatment may be provided by an apparatus operable to select between operating an external compressive therapy, a positive lung pressure therapy, a medicated aerosol therapy, or any combination of the therapies.

The invention at least in the preferred embodiments provides an apparatus which is capable of delivering a number of different breathing therapies, as well as delivering concurrent or contiguous therapies, thereby eliminating the need for multiple devices and separate therapies, but which is also cost effective and does not significantly increase the time required to train operators in its use.

The invention will now be further described by way of example with reference to the accompanying drawings, in which:

FIG. 1 is a diagrammatic view of one embodiment of the composite lung therapy apparatus according to the present disclosure; and

FIG. 2 is a perspective view of one embodiment of an injector nozzle.

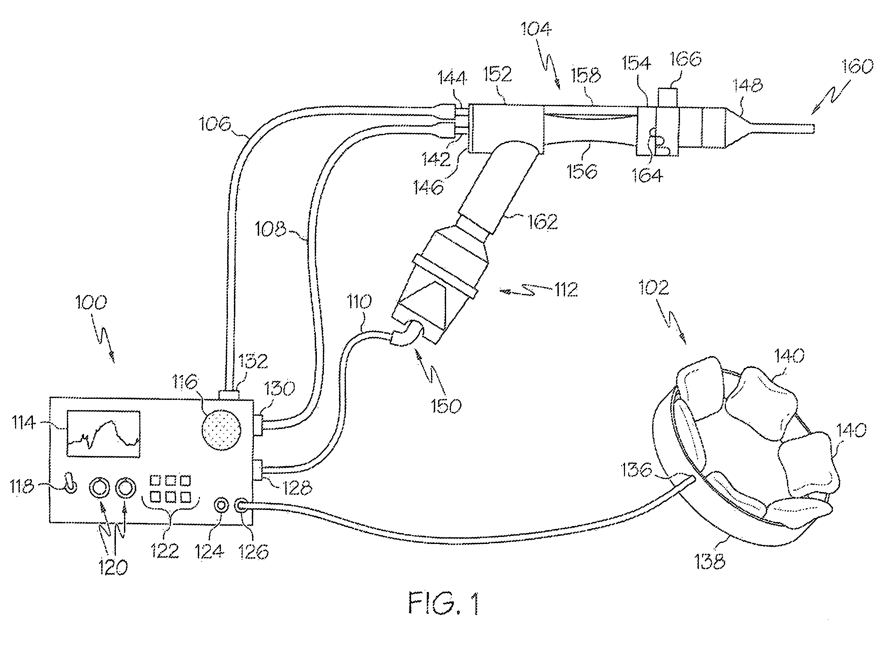

FIG. 1 shows a schematic diagram of an embodiment of the composite lung therapy breathing treatment apparatus comprising a gas control box 100, a chest band 102, and a patient interface circuit comprised of a breathing head assembly 104, circuit tubes 106, 108 and 110, and a nebulizer 112. Circuit tubes 106, 108 and 110 are preferably a small bore (1/8" ID) tube, but as one skilled in the art will appreciate other dimensions may be used.

Gas control box 100 is depicted with a display 114 and a speaker 116. Display 114 may be any visual indicator, such as an indicator light, an analog screen, a digital screen, a mechanical gauge, printed material, and/or any other known or yet to be discovered display. Speaker 116 is shown to represent an audible output device. As one skilled in the art will recognize, any audible output device, such as a bell, buzzer or other mechanical or electrical audible output device, may be incorporated instead of or in combination with speaker 116. Display 114 and speaker 116 may provide system alerts, timer capabilities, and/or patient information entered in the system or monitored by the system.

Gas control box 100 also includes user controls, depicted as switch 118, knobs 120, and buttons 122 in the illustrated example. As one skilled in the art will recognize, user controls may include switches, knobs, buttons, touch-screen displays, voice-activated switches or functions, and/or any other known or yet to be discovered type of control device. In addition, the number, arrangement, and type of user control(s) may vary.

Gas control box 100 also includes a number of input and/or output couplers or fittings 124, 126, 128, 130, and 132. As one skilled in the art will recognize, the number, arrangement, and type of fittings may vary. For example, gas control box 100 is depicted with fittings 124 and 126 in the front, fittings 128 and 130 in the side, and fitting 132 on the top. In the depicted embodiment, fittings 128, 130, and 132 are in operable connection with the patient interface circuit, fitting 126 is in operable connection with chest band 102, and fitting 124 is available for connection to an additional device. Each fitting 124, 126, 128, 130, and 132 may be associated with a dedicated purpose or capable of use for multiple purposes, and the following descriptions of specific purposes for the various fittings are intended as examples associated with the embodiment depicted

Fitting 126 of gas control box 100 is connected to chest band 102 at chest band fitting 136 via chest band tube 134. Chest band 102 is depicted as a circular band 138 with a set of inflatable bladders 140 attached to the interior side of chest band 138. Chest band 138 is intended to fit around the chest of a patient to provide external compressions to assist in and/or facilitate breathing of the patient. As one skilled in the art will recognize, chest band designs and apparatuses other than chest band 102 depicted in this embodiment and other external compressive breathing aides may be used. For one example, a chest band with a single inflatable bladder instead of the multiple bladders 140 depicted may be used to provide the desired oscillating chest compressions. In other embodiments, a vest having one or more air bladders is provided in lieu of chest band 102.

During operation, chest band 102 is secured around the chest of a patient. Once secured, bladders 140 are inflated to an appropriate pressure to compress the chest wall causing a patient to exhale, then bladders 140 are deflated allowing decompression of the chest cavity and the patient to inhale. Bladders 140 continue to inflate and deflate creating an oscillation between the compression and decompression of the chest cavity and the associated exhalation and inhalation of the patient. Gas control box 100 may provide a linear and/or an interrupted gas flow to bladders 140. The compressive pressure may be monitored at chest band 102, at gas control box 100, and/or by a separate pressure monitoring device. In one embodiment contemplated by the present disclosure, gas control box 100 outputs gas via fitting 126 into bladders 140 until the desired upper pressure is achieved, then gas control box 100 removes gas from bladders 140 until a desired lower pressure is reached and repeats the process. In another embodiment according to this disclosure, gas control box 100 outputs gas to pressurize bladders 140, then, once an upper pressure is achieved, a gas release valve provides controlled decompression of bladders 140 until the lower pressure is reached. When the lower pressure is reached the gas release valve closes causing bladders 140 to re-pressurize using the gas flow from gas control box 100. As one having skill in the art will recognize, a gas release valve may be located in a variety of locations in operable association with bladders 140, including incorporated with chest band 102 or gas control box 100.

In the illustrated embodiment, gas control box 100 is also in operable connection with a patient interface circuit comprising a breathing head assembly 104, circuit tubes 106, 108 and 110, and a nebulizer 112 as previously mentioned. Circuit tube 110 connects by one end to fitting 128 of gas control box 100 and by the other end to nebulizer 112. Fitting 130 connects to one end of circuit tube 108, and the other end of circuit tube 108 connects to pulsating gas input 142 in the rearmost end of breathing head assembly 104. Circuit tube 106 also connects to pressure monitoring port 144 in the rearmost end of breathing head assembly 104.

The patient interface breathing head assembly 104 depicted comprises injection nozzle 142, mouthpiece 148, nebulizer 112, pulsating gas input 142, nebulizer gas input 150 and pressure monitoring port 144. In this embodiment, breathing head assembly 104 is shown having a rear cavity 152 associated with a front cavity 154 by means of a venturi tube 156 and pressure monitoring port 144 associated with front cavity 154 by means of feedback tube 158. Mouthpiece 148 and mouthpiece opening 160 are removably attached to the front cavity 154 at the front end of breathing head assembly 104. Nebulizer 112 is removably attached to aerosol entrainment port 162. Aerosol entrainment port 162 is located toward the rear end of breathing head assembly 104, and operatively associated with rear cavity 152. Some embodiments allow for pieces to be removably attached, and any suitable form of removable, permanent, or other type of attachment may be used. As one having skill in the art will appreciate, many suitable attachment means are known in the art.

Circuit tube 110 connects to nebulizer gas input 150 of nebulizer112, which, in turn, connects to aerosol entrainment port 162 located at the rearmost portion of breathing head assembly 104. Circuit tube 108 connects to the rearmost end of breathing head assembly 104 by connecting directly to pulsating gas input 60 which feeds directly into injector nozzle 146. On the side wall of breathing head assembly 104 is at least one forward aperture 164 which opens the front cavity 154 of breathing head assembly 104 to the atmosphere. The forward portion of venturi tube 156 opens into front cavity 154. Feedback tube 158 comprises a first opening adjacent to the forward opening of venturi tube 156 and a second opening that is adjacent to rear cavity 152.

FIG . 2 shows an embodiment of injector nozzle 146, which is inserted into rear cavity 152 at the rear portion of breathing head assembly 104. Adjacent to injector nozzle 146 in this embodiment are a plurality of aft apertures 168 which open breathing head assembly 104 to the ambient atmosphere. Pulsating gas input 142 is shown at the rear of injector nozzle 146. This figure also shows pressure monitoring port connection 170 at the top of injector nozzle 146.

Operation of the patient interface circuit begins by loading a predetermined liquid medicament into nebulizer 112 by first detaching it from aerosol entrainment port 162. After filled nebulizer 112 is reattached, therapy is initiated through the user controls of the gas control box 100. Gas travels from gas control box 100 via fitting 128 through circuit tube 110 to the bottom of nebulizer 112. Nebulizer 112 converts the liquid medication into aerosol which enters into aerosol entrainment port 162, ushering the aerosol into rear cavity 152 of breathing head assembly 104.

Gas control box 100 outputs a high-frequency gas flow via fitting 130 which travels through circuit tube 108 to pulsating gas input 60. Gas control box 100 may create the high-frequency gas flow by interrupting a steady gas flow at a pre-set rate, a user defined rate, or a rate based upon monitored patient conditions. In some contemplated embodiments, the gas flow is interrupted at a rate of at least 1 hertz and at most 15 hertz and the gas flow becomes pulsatile with a substantially constant pressure amplitude. In addition, a user may be able to define the interrupted rate within a limited pre-set range of rates. For example, a user may be able to define a rate within the range of at least 1 hertz and at most 15 hertz. As will be appreciated by one having skill in the art, circuit connectors or direct connections may be used to connect to gas inputs or exhausts. Here, the high-frequency pulses enter injector nozzle 146 which directs them into the rear opening of venturi tube 156. Simultaneously, the increased velocity resulting from the narrowing of injector nozzle 146 lowers surrounding pressures creating a vacuum effect, first described by Swiss mathematician Daniel Bernoulli in 1738, pulling in or entraining additional gas as well as medicament from nebulizer 112. Second, the friction between the high speed molecules and the adjacent low-speed molecules has the effect of pulling the low-speed gas molecules into the stream of the high speed gas.

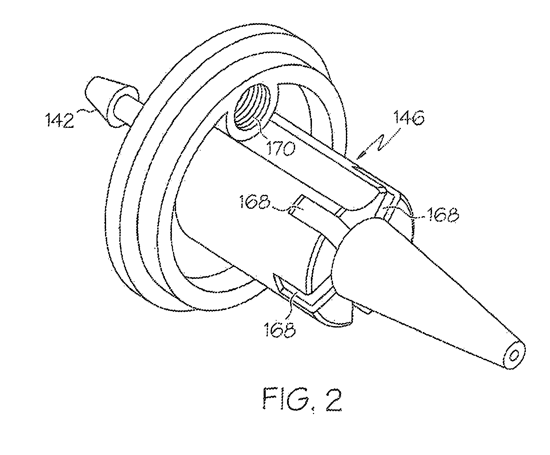

In effect, ambient gas is pulled into the rear cavity of breathing head assembly 104 through aft apertures 168 (shown in Fig. 2) and aerosol entrainment port 162. As the velocity of the gas increases, the volume of entrained gas increases, and, therefore, overall flow increases.

The continuous high-frequency pulsatile flow enters into venturi tube 156 which may either amplify or attenuate it. As the flow enters venturi tube 156, given little or no resistance at mouthpiece opening 160, the flow is amplified. However, as resistance at mouthpiece opening 160 increases, such as would result from the backpressure caused by a patient exhaling into mouthpiece opening 160, the entrainment process is impeded and overall flow is attenuated. Velocity within the venturi decreases, and, in turn, entrainment and flow both decrease. Thus, the device allows the patient to exhale back into it, and the device is provided with a built-in safety mechanism. As the patient exhales or airway compliance decreases, resistance downstream from the venturi tube increases. The resulting decrease in delivered flow also decreases pressure, thereby protecting the airways and allowing the patient to exhale. As one skilled in the art will recognize, mouthpiece 148 may be replaced by a breathing mask, endotracheal tube, or any other known or yet to be discovered means to couple a patient's airways to the patient interface circuit.

The mixture of high-frequency pulsatile flow from injection nozzle 146, aerosol from aerosol entrainment port 162, and ambient air entrained from aft apertures 168 (shown in Fig. 2) continues through the lumen of venturi tube 156, exiting its forward opening into mouthpiece 148 and out mouthpiece opening 160 to the patient. The patient seals his or her lips around mouthpiece 148 and inhales the aerosolized pulses of gas, taking them deep into the lungs. The patient then exhales back into mouthpiece opening 160 as the therapy continues. The combination of aft apertures 168 (shown in Fig. 2) and forward aperture 164 allow both ingress and egress of flow, serving both inhalation and exhalation without the need for complex mechanisms to open and close valves during the therapy.

As the patient continues the high-frequency oscillation breathing therapy, several processes may begin. The medicated aerosol and the oscillation of the air column in the conducting airways may help reduce viscosity of the secretions. The bilevel flow created by high-frequency intermittent delivery of gas may also begin to create wind shear forces. Specifically, during the therapy a small pulse enters the airways, and then the flow momentarily stops. During this pause, the pressure in the upper airways drops. Small volumes of gas that were previously delivered into the airways now begin to exit, momentarily unencumbered by the low pressure condition in the upper airways. As these exiting volumes of gas increase in velocity, they continually push secretions from small distal airways to the larger openings in the upper airways where they can be more easily removed.

Throughout the high-frequency oscillation therapy session, the intermittent positive-pressure pulses continue as the patient inhales and exhales through mouthpiece opening 160. The exhaled breath travels from mouthpiece opening 160 into front cavity 154 and exits forward aperture 164. Aft apertures 168 (shown in Fig. 2) and forward aperture 164 are calibrated with the interrupted rate of flow provided by gas control box 100 so that the patient is able to exhale back into mouthpiece opening 160 even as the high-frequency positive gas flow continues. This calibration allows ample opportunity for exhaled breath to escape in order to prevent the successive stacking of inhaled breaths in the airways.

During the high-frequency oscillation therapy session, the patient's lung pressure may be monitored by attaching a pressure sensor such as a manometer to pressure monitoring port 144 which is associated with the second opening of feedback tube 158. The first opening of feedback tube 158 opens into front cavity 154 and is operatively associated with mouthpiece 148 to improve lung pressure monitoring. The illustrated embodiment is shown with feedback tube 158 having a first opening near mouthpiece 148 to improve the accuracy of the pressure reading and a second opening adjacent to rear cavity 152 to improve the ease of patient handling. As one skilled in the art will appreciate, other positions of a feedback tube may be employed and the feedback tube may be used as a connecting piece. The second opening of feedback tube 158 is operably associated with pressure monitoring port 144. In this embodiment, pressure monitoring port 144 is connected to gas control box 100 via circuit tube 106 and fitting 132. Gas control box 100 monitors the gas flow input through fitting 132. In this embodiment, the monitored gas flow input reflects the pattern of a patient's breathing cycle which gas control box 100 illustrates on display 114.

Collar 166 may be placed over forward aperture 164 to partially occlude forward aperture 164 and affect the ingress and egress of air from front cavity 154. The rotation of collar 166 may be used to allow a variable occlusion to occur over forward aperture 164 depending on the amount of rotation which brings differently sized notches or openings in collar 166 into registry and communication with opening 164 in front cavity 154.

The body of breathing head assembly 104 may be made of a unitary construction that incorporates a first cavity, which in the embodiment shown is front cavity 154, a second cavity, which in the embodiment shown is rear cavity 152, a first passageway, which in the embodiment shown is venturi tube 156, a second passageway, which in the embodiment shown is feedback tube 158, forward aperture 164, and an entrainment cavity, which in the embodiment shown is aerosol entrainment port 162. While unitary construction is used in some embodiments according to the present disclosure, it will be appreciated by one having skill in the art, unitary construction is not necessary.

As one skilled in the art will recognize, although the described operation of the patient interface circuit provides a medicated aerosol treatment therapy, the therapy may be administered without medicated aerosol. For example, nebulizer 112 may be removed allowing aerosol entrainment port 162 to be open to the ambient atmosphere allowing ingress and egress of air. Alternatively, nebulizer 112 may be removed and aerosol entrainment port 162 capped off preventing aerosol entrainment port 162 from being open to the ambient atmosphere. As another alternative, nebulizer 112 may contain a non-medicated water-based solution that provides humidity to the gas flow to help prevent drying of the patient's airways during respiratory treatment.

Additionally, the high-frequency gas flow described herein is for illustrative purposes of the given embodiment. As one skilled in the art will recognize, respiratory therapies may incorporate or use a variety of pulsatile and/or linear gas flow rates.

The user controls may be used to control the parameters of the given respiratory treatment. For example, a user may set the upper pressure and lower pressure for compressions to be applied to the patient with chest band 102. Additionally, the user may set an oscillation rate to administer the chest band compressions, any desired alert conditions, time limits for compression therapy and/or any other parameters for compression therapy. Additionally, the user may set an interrupted rate of gas flow, feedback airflow monitoring parameters, time limits, and other parameters for the patient interface circuit, In addition, the user may set the compression therapy and the patient interface circuit to be administered concurrently. Alternatively, the compression therapy may be set to be administered for a given amount of time at which the compression therapy stops and the therapy through the patient interface circuit immediately begins. As one skilled in the art will recognize, the timing and overlap of any of the respiratory therapies may be set by the user controls and may vary in any number of ways. In addition, as one skilled in the art will recognize, the user may be any person operating the device Including a patient, medical personnel, and others acting on behalf of a patient.
Although certain illustrative embodiments have been described in detail above, many embodiments, variations and modifications are possible.

## Claims

1. A breathing treatment apparatus comprising:
a source of gas;
a patient interface circuit coupled to the source of gas of the gas control box via the first output (128, 130), the patient interface circuit having a breathing head assembly (104) through which the patient exhales and inhales, the breathing head assembly having at least one aperture (164, 168) open to the ambient to allow Ingress and egress of flow, wherein said at least one aperture (164, 168) Is calibrated to allow patient exhalation and prevent stacking of successive volumes of gas in the airway of the patient, the breathing head assembly (104) having a venturi tube (158) fluidly connected to said at least one aperture(164, 168), and an adjuster (166) to partially occlude said at least one aperture (164, 168); **characterized in that** the apparatus further comprises:
a gas control box (100) having the source of gas operable to provide a substantially continuous positive gas flow from the gas control box (100) through a first output (128, 130) of the gas control box, a regulator to regulate the flow of said continuous positive gas flow between said source of gas and a patient, and a selector to select between modes of operation, wherein said modes of operation comprise at least a first mode of operation and a second mode of operation by which different respiratory therapies are provided to the patient's lungs through the patient's mouth; and
at least one inflatable bladder (140) coupled to the gas control box (100) through a second output (126) of the gas control box (100) and placed adjacent a patient's chest, the at least one inflatable bladder (140) being repetitively inflated and deflated by the gas control box (100).

2. The apparatus of claim 1, wherein the breathing head assembly (104) further comprises a nebulizer (112) and an aerosol entrainment port (162) fluidly connectable to said nebulizer for entrainment of aerosol.

3. The apparatus of either claim 1 or claim 2, wherein, in the first mode of operation, the continuous positive gas flow is interrupted at a rate of at least 1 hertz and at most 15 hertz whereby the gas flow becomes pulsatile with a substantially constant pressure amplitude.

4. The apparatus of any preceding claim, wherein the patient is provided with interrupted gas flow in at least one of said modes of operation.

5. The apparatus of any preceding claim, wherein the patient is provided with continuous positive gas flow in at least one of said modes of operation.

6. The apparatus of any preceding claim, wherein said source of gas under pressure comprises an electric compressor.

7. The apparatus of any preceding claim, further comprising a means for measuring pressure.

8. The apparatus of any preceding claim, wherein the gas control box comprises a display (114).

9. The apparatus of any preceding claim, wherein the at least one inflatable bladder (140) Is included as part of a chest wall band (138) that promotes oscillation of the patient airways when said chest wall band is circumferentially wrapped around a patient's chest and a pulsating gas flow is directed to said at least one inflatable bladder of the chest wall band (138).

10. The apparatus of any preceding claim, wherein said patient interface circuit comprises a port (132) connectable to a pressure manometer.

11. The apparatus of any preceding claim, wherein the means for interrupting further comprises an adjustment means to vary the rate at which the flow of said continuous positive gas flow is interrupted.

12. The apparatus of any preceding claim, further comprising a means to prevent inadvertent occlusion of said at least one aperture.

13. The apparatus of any preceding claimfurther comprising a means for tracking use of said apparatus, whereby said patient's breathing therapy can be monitored.

14. The apparatus of any preceding claim, wherein said patient interface circuit is operably connected with a ventilator circuit.

15. The apparatus of any preceding claim, wherein said patient interface circuit is for single patient use.

## Patentansprüche

1. Beatmungsapparat mit:
einer Gasquelle;
mit einer Patientenschnittstellenschaltung, die mit der Gasquelle des Gassteuergeräts über den ersten Ausgang (128, 130) verbunden ist, wobei die Patientenschnittstellenschaltung ein Beatmungskopfteil (104) umfasst, durch das der Patient aus- und einatmet, wobei das Beatmungskopfteil mindestens eine Öffnung (164, 168) aufweist, welche zur Umgebung hin offen ist, um einen Eintritt und Austritt oder Durchfluss zu ermöglichen, wobei die mindestens eine Öffnung (164, 168) so kalibriert ist, dass der Patient ausatmen kann und ein Stau aufeinanderfolgender Gasvolumen in den Luftwegen des Patienten verhindert wird, wobei das Beatmungskopfteil (104) ein mit der mindestens einen Öffnung (164, 168) fluiddurchlässig verbundenes Venturi-Rohr (156) und ein Einstell-Element (160) zum teilweisen Schließen der mindestens einen Öffnung (164, 166) umfasst; **dadurch gekennzeichnet, dass** der Apparat des Weiteren versehen ist:
mit einem Gassteuergerät (100) mit der Gasquelle, die benutzt werden kann, um einen weitgehend kontinuierlichen Gasdurchfluss unter Überdruck aus dem Gassteuergerät (100) durch einen ersten Ausgang (128, 130) des Gassteuergeräts zu bewirken, mit einem Regler zur Regelung des Durchflusses des unter Überdruck kontinuierlich erfolgenden Gasdurchflusses zwischen der Gasquelle und einem Patienten und mit einem Wahlachafter zur Wahl zwischen verschiedenen Betriebsarten, wobei zu den Betriebsarten mindestens eine erste Betriebsart und eine zweite Betriebsart gehören, mit denen verschiedene Beatmungstherapien der Lungen eines Patienten über den Mund des Patienten durchgeführt werden; und
mit mindestens einem aufblasbaren Balg (140), der mit dem Gassteuergerät (100) über einen zweiten Ausgang (126) des Gassteuergeräts (100) verbunden und in der Nähe des Brustkorbs eines Patienten platziert ist, wobei der mindestens eine aufblasbare Balg (140) durch das Gassteuergerät (100) sich ständig wiederholend aufgeblasen bzw. entlüftet wird.

2. Apparat nach Anspruch 1, wobei das Beatmungskopfteil (104) des Weiteren einen Vernebler (112) und einen Aerosolmitnahmekanal (162) umfasst, der zur Mitnahme des Aerosols mit dem Vernebler flulddurchlässig verbunden ist

3. Apparat nach entweder Anspruch 1 oder Anspruch 2, wobei in der ersten Betriebsart der unter Oberdruck kontinuierlich erfolgende Gasdurchfluss mit einer Frequenz von mindestens 1 Hertz und maximal 15 Hertz unterbrochen wird, so dass der Gasdurchfluss mit einer weitgehend konstanten Druckhöhe pulsierend wird.

4. Apparat nach irgendeinem der vorhergehenden Ansprüche, wobei der Patient bei mindestens einer der Betriebsarten mit einem unterbrochenen Gasdurchfluss versorgt wird.

5. Apparat nach irgendeinem der vorhergehenden Ansprüche, wobei der Patient bei mindestens einer der Betriebsarten unter überdruck mit einem kontinuierlichen Gasdurchfluss versorgt wird.

6. Apparat nach irgendeinem der vorhergehenden Ansprüche, wobei die unter Druck stehende Gasquelle einen elektrischen Verdichter umfasst.

7. Apparat nach irgendeinem der vorhergehenden Ansprüche, mit des Weiteren einer Vorrichtung zur Messung des Drucks.

8. Apparat nach irgendeinem der vorhergehenden Ansprüche, wobei das Gassteuergerät mit einem Display (114) ausgestattet ist.

9. Apparat nach irgendeinem der vorhergehenden Ansprüche, wobei der mindestens eine aufblasbare Balg (140) als Teil eines Brustwandbandes (138) ausgebildet ist, welches die Schwingung der Luftwege des Patienten fördert, wenn das Brustwandband um den Brustkasten eines Patienten gelegt ist und ein pulsierender Gasdurchfluss zu dem mindestens einen aufblasbaren Balg des Brustwandbandes (138) erfolgt.

10. Apparat nach irgendeinem der vorhergehenden Ansprüche, wobei die Patientenschnittstellenschaftung eine Öffnung (132) umfasst, an die ein Manometer angeschlossen werden kann.

11. Apparat nach irgendeinem der vorhergehenden Ansprüche, wobei die Unterbrechervorrichtung des Weiteren eine Einstellvorrichtung umfasst, um die Frequenz zu verändern, mit welcher der Durchfluss des unter Überdruck kontinuierlich erfolgenden Gasdurchflusses unterbrochen wird.

12. Apparat nach irgendeinem der vorhergehenden Ansprüche, mit des Weiteren einer Vorrichtung, um ein unabsichtliches Schließen der mindestens einen Öffnung zu verhindern.

13. Apparat nach irgendeinem der vorhergehenden Ansprüche, mit des Weiteren einer Vorrichtung zur Verfolgung der Benutzung des Apparats, so dass die Atemtherapie des Patienten überwacht werden kann.

14. Apparat nach irgendeinem der vorhergehenden Ansprüche, wobei der Patientenschnittstellenschaltung funktionell mit einem Ventilatorschaltkreis verbunden ist.

15. Apparat nach irgendeinem der vorhergehenden Ansprüche, wobei die Patientenschnittstellenschaltung zur Benutzung für nur einen Patienten konzipiert ist.

## Revendications

1. Appareil de traitement respiratoire comprenant :
une source de gaz ;
un circuit d'interface de patient couplé à la source de gaz de la boite de commande de gaz via la première sortie (128, 130), le circuit d'interface de patient ayant un ensemble de tête respiratoire (104) à travers laquelle le patient exhale et inhale, l'ensemble de tête respiratoire ayant au moins une ouverture (164, 168) ouverte à l'air ambiant pour permettre l'entrée et la sortie du flux, dans lequel ladite au moins une ouverture (164, 168) est calibrée pour permettre l'exhalation du patient et empêcher l'empilement de volumes successifs de gaz dans les voies respiratoires du patient, l'ensemble de tête respiratoire (104) ayant un tube de Venturi (156) raccordé de manière fluide à ladite au moins une ouverture (164, 168), et un dispositif d'ajustement (166) pour boucher partiellement ladite au moins une ouverture (164, 168) ; **caractérisé en ce que** l'appareil comprend en outre :
une boîte de commande de gaz (100) ayant la source de gaz pouvant fonctionner pour fournir un flux de gaz positif sensiblement continu à partir de la boîte de commande de gaz (100) en passant par une première sortie (128, 130) de la boîte de commande de gaz, un régulateur pour réguler le flux dudit flux de gaz positif continu entre ladite source de gaz et un patient, et un sélecteur pour choisir entre des modes de fonctionnement, dans lequel lesdits modes de fonctionnement comprennent au moins une premier mode de fonctionnement et un second mode de fonctionnement grâce auxquels on propose des thérapies respiratoires différentes pour les poumons du patient par la bouche du patient ; et
au moins une vessie gonflable (140) couplée à la boîte de commande de gaz (100) par une seconde sortie (126) de la boite de commande de gaz (100) et placée de manière adjacente à la poitrine d'un patient, la au moins une vessie gonflable (140) étant gonflée et de dégonflée de manière répétitive par la boîte de commande de gaz (100).

2. Appareil selon la revendication 1, dans lequel l'ensemble de tête respiratoire (104) comprend en outre un nébuliseur (112) et un orifice d'entraînement d'aérosol (162) pouvant être raccordé de manière fluide audit nébuliseur pour l'entraînement d'aérosol.

3. Appareil selon la revendication 1 ou la revendication 2, dans lequel, dans le premier mode de fonctionnement, le flux de gaz positif continu est interrompu à un débit d'au moins 1 hertz et au maximum de 15 hertz, moyennant quoi le flux de gaz devient pulsatile dans une amplitude de pression sensiblement constante.

4. Appareil selon l'une quelconque des revendications précédentes, dans lequel le patient dispose du flux de gaz interrompu dans au moins l'un desdits modes de fonctionnement.

5. Appareil selon l'une quelconque des revendications précédentes, dans lequel le patient dispose du flux de gaz positif continu dans au moins l'un desdits modes de fonctionnement.

6. Appareil selon l'une quelconque des revendications précédentes, dans lequel ladite source de gaz sous pression comprend un compresseur électrique.

7. Appareil selon l'une quelconque des revendications précédentes, comprenant en outre des moyens pour mesurer la pression.

8. Appareil selon l'une quelconque des revendications précédentes, dans lequel la boite de commande de gaz comprend un affichage (114).

9. Appareil selon l'une quelconque des revendications précédentes, dans lequel la au moins une vessie gonflable (140) est incluse comme faisant partie d'une bande de paroi thoracique (138) qui favorise l'oscillation des voies respiratoires du patient lorsque ladite bande de paroi thoracique est enroulée de manière circonférentielle autour de la poitrine d'un patient et que le flux de gaz pulsé est dirigé vers ladite au moins une vessie gonflable de la bande de paroi thoracique (138).

10. Appareil selon l'une quelconque des revendications précédentes, dans lequel ledit circuit d'interface de patient comprend un orifice (132) pouvant être raccordé à un manomètre de pression.

11. Appareil selon l'une quelconque des revendications précédentes, dans lequel les moyens d'interruption comprennent en outre des moyens de réglage pour modifier le débit auquel le flux dudit flux de gaz positif continu est interrompu.

12. Appareil selon l'une quelconque des revendications précédentes, comprenant en outre des moyens pour empêcher l'occlusion accidentelle de ladite au moins une ouverture.

13. Appareil selon l'une quelconque des revendications précédentes, comprenant en outre des moyens pour suivre l'utilisation dudit appareil, moyennant quoi ladite thérapie respiratoire du patient peut être surveillée.

14. Appareil selon l'une quelconque des revendications précédentes, dans lequel ledit circuit d'interface de patient est raccordé de manière opérationnelle avec un circuit de ventilateur.

15. Appareil selon l'une quelconque des revendications précédentes, dans lequel ledit circuit d'interface de patient est prévu pour l'utilisation par un seul patient.
